# EUROPEAN PATENT SPECIFICATION

(11) **EP 3 307 324 B1**
(45) Date of publication and mention of the grant of the patent: **09.02.2022**
(21) Application number: 16739569.8
(22) Date of filing: 15.06.2016
(51) Int. Cl.: A61K 45/06, A61K 36/9066, A23L 33/00

(54) **FOOD SUPPLEMENT FOR USE IN A PROCESS OF METABOLIC REBALANCING**
NAHRUNGSERGÄNZUNG ZUR VERWENDUNG IN EINEM VERFAHREN FÜR METABOLISCHE REBALANCE
COMPLÉMENT ALIMENTAIRE POUR UTILISATION DANS UN PROCESSUS DE RÉÉQUILIBRAGE MÉTABOLIQUE

(30) Priority: 15.06.2015 IT UB20151383
(43) Date of publication of application: 18.04.2018
(73) Proprietor: Innovitas Vitae S.r.l., 25125 Brescia (IT)
(72) Inventor: MARIOTTI, Massimo, 21040 Gerenzano (Varese) (IT); SCOZZESI, Alesandro, 21040 Gerenzano (Varese) (IT); COLOGNATO, Renato, 21040 Gerenzano (Varese) (IT); MANGANINI, Massimiliano, 21040 Gerenzano (Varese) (IT); PIGOLI, Giuseppe, 21040 Gerenzano (Varese) (IT)
(74) Representative: Zaccaro, Elisabetta
(86) International application number: PCT/IB2016/053533
(87) International publication number: WO 2016/203392

(56) References cited:
- EP-A2- 2 382 978
- WO-A1-2014/184246
- WO-A1-2014/191447
- WO-A1-2015/055468
- WO-A1-2015/057751
- WO-A2-01/78783
- US-A1- 2002 034 555
- US-A1- 2008 026 983
- US-A1- 2014 329 913
- US-A1- 2015 086 484
- G SHOBA ET AL: "Influence of piperine on the pharmacokinetics of curcumin in animals and human volunteers", PLANTA MED., vol. 64, no. 4, 1 January 1998 (1998-01-01), pages 353-356, XP055218622,
- PATHAK ET AL: "Cytoprotective and immunomodulating properties of piperine on murine splenocytes: An in vitro study", EUROPEAN JOURNAL OF PHARMACOLOGY, ELSEVIER SCIENCE, NL, vol. 576, no. 1-3, 19 October 2007 (2007-10-19), pages 160-170, XP022307552, ISSN: 0014-2999, DOI: 10.1016/J.EJPHAR.2007.07.033
- RAGHVENDRA M SRIVASTAVA ET AL: "Immunomodulatory and therapeutic activity of curcumin", INTERNATIONAL IMMUNOPHARMACOLOGY, ELSEVIER, AMSTERDAM, NL, vol. 11, no. 3, 22 August 2010 (2010-08-22), pages 331-341, XP028152235, ISSN: 1567-5769, DOI: 10.1016/J.INTIMP.2010.08.014 [retrieved on 2010-08-27]

## Description

### Technical field

The present invention relates to a composition of substances defining a so-called "food supplement" which can be used to induce, in a body that takes it in, biochemical processes modifying metabolic and/or physiological cycles within the body itself.

### Prior art

As is well known, various chemical substances of natural or synthetic origin can provoke alterations of the metabolic equilibrium in bodies, inducing non-physiological states, and it is likewise known that other substances can counter the action of harmful agents (exogenous or endogenous) introduced into the body: in this regard, one may mention the so-called "anti-oxidants" present in various types of edible substances of natural origin, e.g. anthocyanins, polyphenols, carotenoids, various types of vitamins (tocopherols and tocotrienols, ascorbic acid), as well as in endogenous enzymes with an anti-oxidant action, such as glutathione, catalase, superoxide-dismutase and various peroxidases.

In general, therefore, it is known that a body can take in different substances contained in natural (or even synthetic) foods which have functions of countering the metabolic alterations produced by one or more factors that are harmful (toxic) for all organic molecules in general and thus also for the cellular component. There are thus various known types of "strategies of assimilation" of these substances, aimed at regulating physiological activities in an optimal manner (in particular to combat so-called "oxidative stress/damage" and the pathologies correlated therewith): the methods of intervention in the body usually provide for the intake of appropriate compounds, which are then absorbed by the digestive system and afterwards distributed in the body, where, finally, they exert their effects in a more or less intense manner.

The compositions of a known type are not, however, free of drawbacks, particularly in terms of effectiveness of absorption of the substances ingested and the consequent effect on physiological conditions: not infrequently, in fact, active principles that are theoretically effective in laboratory conditions are almost insignificant or inactive under real conditions of use and assimilation (in such cases, one speaks of problems tied to so-called "bioavailability").

Another problematic aspect of the known compositions lies in the fact that in the combinations of various food substances (which in turn deliver the active ingredients to the body during ingestion and the subsequent assimilation), the active ingredients prove to be not only scarcely bioavailable, but can also prove to be scarcely assimilable by the body: this makes it rather difficult to identify the correct amounts of composition and also gives rise to a risk of under- or overdosage.

Moreover, the formulations of food supplements of a known type, despite being constructed on the basis of well-established, scientifically documentable principles of non-toxicity, are in general conceived to have a generic effect on the body, and do not "target" particular dysfunctions: this forces one to proceed on a trial-and-error basis in "gentle" treatments of physical malaise, since it is necessary to try out - over what may also be a particularly long time - different "recipes" if it is desired to verify effectiveness against a particular state of physical malaise.

US2008/026983 discloses a nutritional composition for reducing inflammation, comprising curcumin and bromelain.

US2002/034555 discloses a medicinal composition for treating migraine headache pain, comprising bromelain and curcumin.

Shoba G. et al. ("Influence of piperine on the pharmacokinetics of curcumin in animals and human volunteers" Planta Med 1998 May;64(4):353-6) and Pathak and Khandelwal ("Cytoprotective and immunomodulating properties of piperine on murine splenocytes: an in vitro study" Eur J Pharmacol, 2007 Dec 8;576(1-3):160-70) relate to the effects of piperine on immunomodulation and bioavailability.

### Detailed description of the invention

In consideration of the prior art and the problems mentioned here above, the object of the present invention is thus to provide a composition of substances, definable as a constituent base of a food supplement, which is able to remedy the drawbacks of the prior art.

More particularly, the object of the present invention is to implement a composition that is highly efficient in terms of both bioavailability and assimilability, thus being much more suitable for being ingested or in any case assimilated (and being capable of exerting a more "intense" function on the body).

The present invention also has the object of devising and preparing - also on an industrial scale or in any case with high production volumes - a composition for a food supplement that is simplified to the utmost in terms of variety and/or relative amounts of the individual components/ingredients, and is in any case highly effective in terms of the assimilability and ready availability of its "active ingredients" in those regions of the body that require the actual localized action thereof.

From the viewpoint of formulation flexibility, the present invention also has the object of being able to offer different composition variants, which all have in common, however, a very high bioavailability of the functionally most important active ingredients and a rapid assimilability also by subjects having different metabolism/digestion/assimilation capacities or who are even compromised by more or less severe pathological conditions.

The object of the invention is also to considerably improve the actual operational capability of the composition, making it suitable for intervening in specific conditions, and in particular conditions of toxicity/intoxication (or also, according to a term in current jargon in natural medicine or naturopathy, in conditions of so-called "intoxination") that are equally peculiar, e.g. conditions of toxicity/activity of the so-called "blood complement", which in turn are correlatable to different types of physical malaise.

Yet another object of the present invention is to devise a food supplement that can be made with accurately determinable amounts of active ingredients, even when based on different commercially available ingredients with a different degree of purity or concentration or titre or in any case a different form of aggregation (powders that are more or less fine/homogeneous, with variable amounts of residual moisture and so forth).

Moreover, a further object of the present invention is to provide a food supplement for use in the treatment and prevention of female infertility, more specifically in the treatment and/or prevention of "unexplained", and in particular immune-mediated, female infertility.

The present invention therefore relates, in a first aspect, to a composition for use in the treatment of immune-mediated female infertility, wherein said composition comprises:
- bromelain, in an amount of at least 215 mg by weight per single unit of administration;
- at least one substance capable of exerting an antioxidant action, preferably selected from among papain, curcumin and mixtures thereof, wherein said antioxidant substance comprises, per single unit of administration:
- papain, in an amount of at least 125 mg by weight; and/or
- curcumin, in an amount of at least 131.5 mg by weight,
characterized in that it further comprises:
- a substance facilitating penetration through a cell membrane; said substance being piperidine.

The present invention relates to the composition as described above for use in the treatment of immune-mediated female infertility.

In other words, the present invention is based on the fact that the substance facilitating cell penetration is active on the anti-oedemigenous and/or anti-inflammatory substance and/or on the antioxidant substance.

In order to be optimally balanced in terms of "concentration" of active substances, the total mass of a single unit of administration of the composition itself can have an overall weight comprised between 0.40 grams and 1 grams and even more preferably comprised between 0.45 grams and 0.8 grams, and the respective optimal amounts of the individual components of the composition can be indicated in relation to this reference amount (which in fact corresponds to that of a single dose that can be taken by a subject).

More particularly, with regard to the so-called penetration facilitating substance, the following types of molecules/substances can be indicated, along with these intervals of weight/presence per single unit of administration:
- membrane phospholipids (present, for example, in an amount of at least 200 mg by weight); and/or
- biopolymers or biocompatible polymers; and/or
- serum albumin and/or bovine serum albumin.

As regards, even more specifically, the possible presence of membrane phospholipids, the latter in turn can comprise phosphatidylinositol and/or phosphatidylethanolamine and/or phosphatidylcholine; and/or phosphatidylserine.

As regards the biopolymers or biocompatible polymers, the latter can comprise polyethylene glycol (known in this technical field by the acronym "PEG") and/or polyamide amine/ polyacrylic acid (known in this technical field by the acronym "PAA") and/or polycaprolactone (known in this technical field by the acronym "PCL") and/or poly(trimethylene carbonate) (known in this technical field by the acronym "PTMC").

As regards the so-called antioxidant substance, it may be seen that, in accordance with the invention, this can comprise (again per single unit of administration) papain, which can be present, for example, at least in 125 mg of weight per single unit of administration and/or curcumin, added to the composition preferably as a dry curcuma extract, which can be present, for example, at least in 131.5 mg of weight per single unit of administration. Depending on the degree of effectiveness it is desired to impart to the composition, it is advantageously possible for the curcumin to be present in phytosome form, i.e. in an aggregated/complexed form in which the curcumin is associated with soy phospholipids (such as, for example, phosphatidylcholine): in such a manner one can obtain a bioavailability of the curcumin that is approximately 10 times greater than the bioavailability of pure curcumin.

As regards the anti-oedemigenous and/or anti-inflammatory substance, it may be seen that the composition for use of the present invention comprises the presence of bromelain, as defined by claims 1-9.

As regards the substance facilitating penetration through a cell membrane, the present invention comprises the presence of piperidine, preferably added to the composition in the form of a dry black pepper extract: the dry black pepper extract can in turn be present in an amount by weight of at least 1.05 mg per single unit of administration.

Conveniently, in order to increase speed and effectiveness in terms of absorption by the subject who takes it, this composition can have at least one absorption and bioavailability multiplying substance, which acts at least on the antioxidant substance: the absorption/bioavailability multiplying substance can also coincide with the aforementioned membrane phospholipids, which can thus simultaneously perform a dual function within the composition itself.

From a quantitative viewpoint, in order to perform this simultaneous dual function, the membrane phospholipids can be present in a ratio of 5 to 1 by weight relative to the curcumin (in terms of relative weight within a single unit of administration, already defined previously and in any case claimed hereunder).

Again with respect to the optional components of the present composition, one can apply at least one substance inhibiting cellular metabolic processes on the antioxidant substance: the metabolic processes to be inhibited usually comprise processes of digestion and/or breakdown preceding an absorption of the antioxidant substance through a cell membrane, and are precisely to be avoided in order to preserve the antioxidant substance until it arrives at the "physiological site" where it must perform its primary functions without having been degraded.

Again optionally (but it is also possible for the present invention to perform its peculiar functions even in the complete absence of this substance), the present composition can have at least one enzyme belonging to the hydrolase family and capable of exerting a fibrinolytic and/or proteolytic action (for example, trypsin).

According to a particularly preferred embodiment, the composition according to the invention can comprise:
- curcuma, preferably as a dry extract;
- papain;
- piperidine, preferably as a dry black pepper extract; and
- bromelain, preferably 1,200 GDU.

According to a first variant, said composition can comprise:
20.0 - 30.0% by weight, preferably 26.0 - 29.0%, more preferably approximately 27.8%, of curcuma, preferably as a dry extract;
20.0 - 30.0% by weight, preferably 25.0-28.0%, more preferably approximately 26.5% of papain;
0.1-0.3% by weight, preferably approximately 0.2%, of piperidine, preferably as a dry black pepper extract; and
40.0 - 50.0% by weight, preferably 43.0 - 47.0%, more preferably approximately 45.5%, of bromelain, wherein said bromelain can preferably be bromelain 1,200 GDU,
said percentages referring to the weight of the single component relative to the sum of the weights of the specified components.

According to a further variant, said composition can comprise curcuma, papain, piperidine and bromelain in the above-specified amounts and/or in the following ratios by weight:
- papain/piperidine 110-160 (e.g. 110-160 mg of papain per 1 mg of piperidine);
- bromelain/piperidine 175-225;
- curcuma/piperidine 100-130.

In the preferred embodiment thereof, the composition according to the invention, and in greater detail the combination of substances contained therein, have an effectiveness of use thanks to the synergy of the active ingredients in immunological and inflammatory problems, in relation in particular to the problems of reproductive medicine. The highest effectiveness of the composition according to the invention has been induced, from a biological and clinical pharmacological viewpoint, by the presence of piperidine which, in terms of both quantities (milligrams) and ratios (milligrams) with the other substances, ensures that the mainly antioxidant substance present in the composition, i.e. papain, and bromelain, which tend to have an anti-inflammatory and antibiotic action, manage to act synergically, thanks to their ratios with piperidine, thus increasing the effect and effectiveness and tending to significantly increase the absorption capacity of the aforesaid substances at the intestinal level. In light of this, it is in fact important to underscore that the individual substances, if not included in the concentrations indicated above and in the aforesaid ratios, would not have the same clinical effectiveness since they would not be absorbed in sufficient amounts to induce the effect.

In one embodiment, the present invention relates to a food supplement for use in the treatment of immune-mediated female infertility, comprising the composition as described above and at least one physiologically acceptable excipient.

Said composition and/or said supplement can be in the form of tablets or capsules, preferably gastroresistent, said composition and/or said supplement being able to be prepared by means of processes known to the person skilled in the art, such as, for example, dry mixing.

Going back to the characterization of the various components contributing to form the present composition, it should be noted that, advantageously, the substance with anti-oedemigenous and/or anti-inflammatory action and/or the substance with antioxidant action and/or the penetration facilitating substance can be present - in a single unit of administration - in a micrometric and/or nanometric granular form: in the terms of the present invention, "micrometric and/or nanometric granular form" thus means a micronized granular solid state in which corpuscles chemically defined by these substances typically have an average diameter comprised between 0.01 micrometres (µm) and 2 micrometres (µm) (for example, between 0.3 µm and 1.5 µm).

In order to have the various substances in micrometric and/or nanometric granular form, it is possible to use any production process known today that is suited to this purpose, such as, for example, the currently known and most widely used "mill" processes/machinery, or also more technologically advanced processes/machinery (such as, for example, micronizers which exploit laser radiation and/or so-called supercritical fluids-based micronizers).

If it is necessary to impart additional functions to the present composition, a means for regulating an activity of immune cells of the "Th1 " and/or "Th2" type can further be present: such regulating means are of a known type and can be found in the technical documentation of the sector concerned.

From the viewpoint of practical applications, the composition of the present invention is based on the synergy of the respective components and in particular it is based on two prevalent synergic mechanisms, namely:
- the synergy between the bioavailability multipliers and the other categories of substances (which offer, as an overall result, an improvement in the absorption of curcumin thanks to the penetration facilitating substances); and
- the synergy between the substance inhibiting processes of digestion and/or breakdown of the antioxidant substances and the antioxidant substances themselves.

Among the two synergies mentioned, the former brings about a considerable reduction in the intervention times and a corresponding increase in the intensity/measurability of the effects characteristic of the antioxidant substances, whereas the latter prevents the typical metabolic/digestive cycles of a body that takes in the compositions of the present invention "through food" from impairing the efficiency and/or the total amounts of antioxidant substances that "must" reach the area in which their effect is required (in other words, the synergy between the substance inhibiting extra-cellular metabolic processes and the antioxidant substances prevent the curcumin - or papain - from being "predigested/broken down" before being absorbed into cells).

It should moreover be noted, again within the scope of the invention, that the presence of trypsin is not necessary or is in any case optional, so much so that composition formulations that provide for a complete absence thereof are possible: in these formulations without trypsin, the function of this substance is performed by one or more alternative substances conveniently selected from among the ones listed here and in any case claimed hereunder.

The possibility of using these compositions consists mainly in the so-called rebalancing of the metabolic and/or physiological functions: for example, one might think of using this composition to induce a re-modulation (or a re-balancing, using the terminology already introduced previously), within the body, of the so-called "blood complement" functional parameters.

In even greater detail, the present composition can be employed to induce a re-balancing between these blood complement components, which as is known are correlated with a level of activity/toxicity thereof:
- factor H"; and/or
- factor "I"; and/or
- factor "B"; and/or
- factor "D"; and/or
- factor "P".

The intake of the composition according to the invention stimulates an indirect action whereby the body (and in particular the liver of this body) is placed in a condition to produce a new amount of complement that is characterized in that it has absolute and/or relative amounts of the factors "H", "B", "I", "D" and/or P" corresponding to a desired level of activity.

From the viewpoint of the terminology used in the present invention, a definition must be given of the so-called blood complement "activity", i.e. of the actual state of activity and/or intensity with which this complex of substances attacks everything it comes into contact with and which is considered foreign to the body that produced the complement itself.

To complement what has just been described, it may be noted that this composition can be used, for example to supplement a dietary/nutritional regime (or, as more currently expressed, in the application of a nutritional protocol), in order to induce a subject/patient and/or body to produce new amounts of blood complement having levels of activity or toxicity that prevent, for example, the triggering of immune reactions or of pathological mechanisms tied precisely to blood complement activity/toxicity.

This "indirect" approach to body metabolism for which the composition - or rather, compositions - are intended according to the invention is thus based on stimulating one or more of the body's metabolic mechanisms (for example, stimulation of the liver to produce a blood complement with different absolute and/or relative values of the previously mentioned "factors") in such a way as to favour and/or obtain a general rebalancing of toxicity conditions.

According to the needs of the moment, this food supplement can conveniently also induce a decrease in the presence of free radicals (of both oxygen and nitrogen) and stimulate the liver to produce a "new" amount of "blood complement" characterized by a different balance of the factors "B", "H", "D", "P" and "I".

Moreover, the present composition (or in other words, the food supplement based on this same composition) can contribute to regulating other potentially dangerous activities of the immune system, such as, for example, the ratio between the substances/chemical species produced by lymphocytes of the "Th1" type and those produced by lymphocytes of the "Th2" type (for example, cytokines).

The possibility of using the food supplement (in its various formulations) as described thus far and claimed hereunder in a process of metabolic modification aimed at varying the composition of the so-called "blood complement" can be correlated/integrated with an innovative and original method of identifying and countering toxic factors in samples of a preferably organic nature.

Speaking of methods of toxicological analysis, the identification of the chemical/biological substances that can trigger toxic reactions in a body is broad and varied both in terms of experimentation and detection methods and in terms of hypothesizing and verifying cause and effect relationships between substances promoting toxic reactions and "macroscopic" consequences inside the body in which the toxic reactions occur.

For example, there is known in the art a so-called "CH50" test, which is capable of defining so-called "blood complement activity": this test is specifically applied to an individual type of organic sample (which is an appropriate amount of blood) and is thus by its very nature scarcely flexible for other types of examinations.

A further limitation of the "CH50" test is given by the fact that it is capable solely of revealing toxic reactions tied to the intervention of antibodies (definable in general as "antibody-mediated mechanisms") but is not sensitive to all the chemical and biological phenomena whereby the toxic reaction is triggered directly, i.e. without the arrival and attack of antibodies.

Another test of a known type, called "APH50", assesses the activity of the non-antibody-mediated complement: this test uses a so-called "lipopolysaccharide" (a bacterial protein that causes inflammation during infections) as an antigen/inducer and measures the ability of the blood complement to aggregate in attack complexes but not to actually destroy an embryo.

It is also possible for the "APH50" test to use, as a "target", rabbit red blood cells, which are destroyed in amounts proportional to the activation of the blood complement: though the antigens/inducers are more numerous on the membrane of the target cells, this test measures the ability of the blood complement to destroy individual cells, but not embryos.

The methods of a known type are therefore not free of drawbacks, particularly in terms of the accuracy in identifying toxic substances: not infrequently, in fact, in the presence of organic samples that are extremely varied in terms of composition, there coexist therein very diverse molecules or "active elements" (such as particular types of cells with specific functions, protein and/or hormonal complexes or in any case products deriving from metabolic processes) which can cooperate or mutually oppose each other.

It is therefore very difficult to plan a strategy for countering toxic substances unless they are identified with precision.

In consideration of the prior art in respect of methods and the problems mentioned here above, the object of the present invention is therefore to provide a method for identifying and countering toxic factors in samples of a preferably organic nature.

More particularly, the object of the present invention is to implement a method of identifying, as accurately as possible, the substances or in any case the chemical species that trigger given reactions: this accuracy is consequently useful for determining the clearest possible correlation between data typical of pathological (or in any case physiologically) situations and identifying a correct strategy of intervention in order to counter, mitigate or even eliminate the conditions that trigger toxic-pathological reactions.

Yet another object of the present invention is to devise a method that can be sensitive to toxic reactions, promoted by organic substances, which are not mediated by the action of antibodies, and, again from an operational viewpoint, can also be sensitive to all the possible variations of the quantitative or qualitative parameters of the samples analyzed, for example, at different moments in a given time period (daily or also longer) or under different physiological or pathological conditions of a subject from whom the samples are taken.

The present method is moreover aimed at the development of a diagnostic protocol for various pathologies, for example of the "immune" type.

A description will now be provided, by way of non-limiting example, of an embodiment of a method which mainly consists of this sequence of steps:
- preparing a sample of organic material (which is typically from a subject whose physiopathological state is to be analyzed or in whom it is in any case desired to determine the degree of activity or "hazardousness");
- associating, for example with the usual laboratory instruments, a biologically active material with a sample of organic material for a predetermined period of exposure; and
- following said association, proceeding to verify (possible) immune and/or toxic reaction phenomena occurring in the biologically active material during the period of exposure to the sample of organic material.

Advantageously, during the just mentioned verification of the possible occurrence of immune and/or toxic reaction phenomena, a (sub)step of determining a specific toxicity parameter is implemented: this specific toxicity parameter is univocally correlated with a subset of critical chemical substances present in said organic sample (and is thus tied to a much more accurate and precise determination of the actual cause of toxicity of the sample, which generally contains a very high and variable number of different chemical substances).

In greater detail, the method according to the present invention is dedicated to identifying a specific toxicity parameter which is tied to an absolute amount of a single "critical" chemical species.

In any case, again in order to obtain a high causal discrimination of the present method, the specific toxicity parameter is quantifiable and correlatable to a relative ratio (amount) between two or more "critical" chemical substances.

The identification of a single critical substance/species, or in any case of a ratio between a very limited number of substances/species within the organic sample proves to be particularly useful in order to have an indication of the possible immune or toxic reaction processes that take place by interaction between molecules that come into direct reciprocal contact: in other words, the present method envisages the possibility of correlating the specific toxicity parameter to immune or toxic reactions of a type that is not mediated through antibodies.

From the viewpoint of its implementation, the method can conveniently associate the biologically active material with the sample of organic material by exposing the sample of organic material to at least one living organism having an ability to nourish itself autonomously via temporary organs associated with the living organism itself and capable of supplying nutritive substances to the latter without drawing them from an outside environment: this particular choice brings different advantages in terms of the accuracy of the results as to the measurement of the specific toxicity parameter, as well as in terms of repeatability on a vast scale.

In greater detail, the biologically active material can comprise at least one living organism able to nourish itself autonomously via a temporary organ such as a "yolk sac" or of a similar type, such as, for example:
- one or more fish belonging to the species *Danio Rerio*, with a lifetime less than or equal to 120 hours of development and/or post-fertilization cell multiplication; and/or
- one or more fish belonging to the species *Oryzias Latipes*, with a lifetime less than or equal to 288 hours of development and/or post-fertilization cell multiplication; and/or
- one or more amphibians belonging to the genus *Xenopus*, with a lifetime less than or equal to 96 hours of development and/or post-fertilization cell multiplication.

From the viewpoint of the organic sample, the present method has a particularly exemplary application where one chooses the so-called "blood complement": in relation to this type of substance (or rather, in relation to this mix of different physicochemical factors), the substep of determining the specific toxicity parameter is dedicated to establishing a parametric relation with respect to absolute and/or relative amounts univocally associable with the so-called "factor H" and/or so-called "factor B" and/or so-called "factor I" and/or so-called "factor P" and/or so-called "factor D" present in the "blood complement".

From the viewpoint of the terminology used in the present invention, it is thus possible to give a definition of so-called blood complement "activity", i.e. of the actual state of activity and/or intensity with which this complex of substances attacks everything it comes into contact with which is considered foreign to the body that produced the complement itself.

It should be noted that in the case of relative ratios between the presence/amounts of the different types of "factors" present in the blood complement, the present method envisages that the specific toxicity parameter is univocally associable with a ratio between blood complement factors promoting toxic activity (which are typically the factor "P" and/or factor "D" and/or factor "B") and between blood complement factors inhibiting toxic activity (which are typically the "factor H" and/or "factor I").

In relation to the (absolute or relative) quantifications listed here above (and claimed hereunder), it is thus possible to determine specific toxicity parameters and then correlate them with the onset of (ongoing or even predicted!) pathological conditions of varying nature: this suggests that the present invention can also be a method for predicting pathological states and even, by identifying the causes thereof with extreme accuracy and clarity of the cause and effect relationship, enabling a strategy of treatment action to be implemented.

In other words, it is advantageously possible that the present method may also comprise a step of modifying, based on feedback, the specific toxicity parameter, which is reflected in turn in a variation in the absolute or relative amounts of the factors that actually cause toxicity: this makes it possible to eliminate, in an almost complete and targeted manner, the causes of various pathological situations, by eliminating, for example, undesired non-antibody-mediated toxic reactions.

From the viewpoint of implementation, the feedback-based modification of the organic sample can be directly implemented on the organic sample and comprises a set of possible corrective intervention strategies, among which we can mention:
- selectively adding to the organic sample chemical substances capable of compensating for/increasing an absolute amount of the single critical chemical substance tied to the specific toxicity parameter (if the preceding steps have identified/determined a specific toxicity parameter below a threshold value); and/or
- selectively adding chemical substances capable of mitigating/decreasing an absolute amount of the single critical chemical substance tied to the specific toxicity parameter (if, by contrast, the preceding method steps have identified/determined a specific toxicity parameter above a threshold value); and/or
- selectively adding chemical species capable of bringing the quantitative ratio between the critical chemical substances tied to the specific toxicity parameter back near the threshold value.

If these control strategies (or, in other words, of re-modulation of the level of toxicity/activity) are implemented on the blood complement, the possible selective additions can be thus listed:
- direct addition of predetermined compensating amounts of the "factor H"; and/or
- direct addition of predetermined compensating amounts of the "factor I"; and/or
- direct addition of predetermined amounts of substances that inhibit the action of the "factor B" and/or factor "P" and/or factor "D" (such as, for example, the "anti-factor B antibody", which is today commercially available).

The re-modulation intervention on the organic sample can be direct (and can thus be done directly on the organic sample taken and analyzed), and this direct intervention can be replicated, if necessary, on the organic material - and consequently on the body that is producing them and thus hosts them - which actually has characteristics identical to those of the analyzed sample: this therefore suggests the possibility of intervening on a subject/patient after verifying preliminarily and very rapidly the effectiveness of the intervention of re-modulation.

The feedback-based intervention can thus be oriented towards inducing, in the subject/patient and/or body that produces the organic sample, the production of a new amount of organic material (which in turn can be the source of a new organic sample) having a specific re-modulated toxicity parameter, preferably decreased relative to a first specific toxicity parameter measured in a first implementation of the method itself.

From the viewpoint of the synergy between the two inventive aspects presented thus far, it may be seen that the administration of the food composition described above in this text can have a direct effect on the re-modulation of one or more blood complement factors, which can, for example, bring the absolute values (or the relative values, which can also be expressed in terms of mathematical ratios between the absolute values tied to the individual factors) back within levels considered "physiologically healthy", starting from the "abnormal" values previously identified with the method itself.

For example, considering the "blood complement" (a mixture of proteins produced by the liver which usually attack elements considered to be "foreign" to the body through antibody-mediated mechanisms of intervention/reaction and/or non-antibody-mediated mechanisms of reaction/intervention), the above-described detection method can be used to evaluate the so-called embryo toxic factor (ETF), which is in turn correlated to infertility phenomena (failure of the embryo to settle in the endometrium and/or premature death of the embryo itself).

Going back to the concept of blood complement activity, it must be noted that this is a parameter of great importance in the reaction/intervention mechanisms not mediated by the action of antibodies and, again from an operational viewpoint, it must also be pointed out that the level of activity of the complement can vary in a circadian manner or in relation to variations in the physiological or pathological conditions of a given subject: given the extreme variability of this parameter depending on subjective characteristics, it is therefore worth observing that the present method makes it possible to obtain diagnostic analyses that are equally accurate and absolutely "focused" on the conditions of the individual patient/subject/body, and can likewise be used to calculate personalized dosages of "corrective" substances accordingly.

The level of blood complement activity can thus be correlated to a specific toxicity parameter, which, according to the experimental tests performed by the applicant, can be in turn correlated to one or more substances belonging to a very narrow group and present in the blood complement itself: these are the so-called "factor H", the so-called "factor B", the so-called "factor P ", the so-called "factor D" and the so-called "factor I".

From a functional viewpoint, the "factor H" and the "factor I" are inhibitors of blood complement activity, whilst the "factors "B", "D" and "P" are promoters of blood complement activity: in accordance with the present invention, it was verified that the triggering of toxic or immune reactions of the blood complement (e.g. an embryo toxic reaction) can be controlled by decreasing the amount of its promoting factors and/or increasing the amount of its inhibiting factors.

From the viewpoint of practical applications, it is thus conceivable to intervene directly on the blood complement by injecting the missing factors (in the case of low levels of "factor H" and/or factor "I") and/or the inhibitor of the factors "B" and/or "D" and/or "P" directly into it: alternatively, it is conceivable to intervene upstream of the blood complement production process by stimulating an indirect action whereby the body (and in particular the liver) is placed in a condition to produce a new amount of complement which is characterized by having absolute and/or relative amounts of the factors "H", "B", "I", "D" and/or P" corresponding to a desired level of activity or of toxicity.

To complement what has just been described, it may be noted that this method can be used in order to induce a subject/patient and/or body to produce new amounts of blood complement having levels of activity or toxicity that prevent, for example, the triggering of immune reactions or of pathological mechanisms tied precisely to blood complement activity/toxicity.

This "indirect" approach to the metabolism of the subject/patient and/or body can for example provide for the administration of medicines or food supplements which in practical terms act on the overall metabolism of the body in such a way as to favour and/or obtain a general rebalancing of the conditions of toxicity (for example, by stimulating the liver to produce a "new" amount of "blood complement" characterized by a different balancing of the factors "B", "H", "D", "P" and "I").

The composition invention achieves various advantages compared to the prior art.

First of all, it should be pointed out that the peculiar functional combination between substances that improve absorption and bioavailability and substances with antioxidant action or in any case regulating and rebalancing substances (also definable as anti-toxic) considerably amplifies the effect on the body's metabolism and on some of the main systems of self-regulation of the physiological levels of toxicity.

In particular, the present composition is particularly effective in placing the liver in optimal conditions for producing amounts of blood complement that are optimized in terms of the co-presence of the factors "H", "I", "D", "P" and "B".

Moreover, the present composition can also influence the regulation of an activity of immune cells of the "Th1" and/or "Th2" type, of cytokines (it shall be noted in this regard that the factor "P" of the blood complement is also produced by immune cells which are indirectly conditioned and balanced by at least one of the possible composition embodiments according to the invention).

In terms of possible applications/uses, the present composition thus has good specificity in containing and reducing the conditions typical of immune-mediated infertility, and in any case by exploiting its anti-toxic and immune-modulating action it can advantageously be used in a patient/subject/body in order to counter: (not pertaining to the invention)
- cardiocirculatory pathologies; and/or
- rheumatic pathologies; and/or
- pathologies of the upper and/or lower respiratory tracts; and/or
- pathologies of the urinary and/or reproductive systems; and/or
- pathologies of the lymphatic system; and/or
- traumas;
- pathological states ascribable to the family of so-called "inflammatory bowel diseases" (such as, for example, Crohn's disease or ulcerous colitis); and/or
- acute and chronic skin pathologies.

In particular, it was surprisingly found that the composition according to the invention as described above can be used in the treatment of immune-mediated female infertility.

More in general it can thus be noted that the compositions for use described here above (and claimed hereunder) have a very high degree of assimilability thanks to the noteworthy synergic effect developed by the respective main components, while simultaneously offering a broad spectrum of actions aimed at improving the physiological state, thanks to the re-modulation of the levels of blood complement activity/toxicity or also of other negative factors that impair physical wellbeing (for example, the concentration of free radicals).

It is clear, finally, that the subject matter of the present invention, expressed both in terms of method and of compositions that can be produced/used in that method (or in a broader protocol for attaining optimal physiological conditions or personal wellbeing), can undergo additions or modifications that are obvious for the person skilled in the art without going beyond the scope of protection provided by the claims.

The invention relating to the method of measuring toxicity parameters, descriptively introduced above and also implementable as an independent invention, brings various advantages compared to the prior art, as well as having an evident correlation with the composition described and primarily claimed (which can thus be prescribed and/or used precisely after the factors of toxicity have been determined and can therefore be used to determine the best physiological conditions for bringing the factors back to optimal values of the toxicity factors).

First of all, thanks to the highly specific identification of the components or interacting subgroups within the complex masses of chemically active substances, it is possible to determine, with very high accuracy, both the condition of toxicity and the causes triggering it: this makes it possible to perform very accurate diagnoses, also of a predictive type, and also to identify highly targeted strategies of intervention, mainly aimed at rebalancing the amounts of the components that stimulate toxic reactions (bringing them back to absolute or relative amounts equal to or in any case comparable with physiologically acceptable ones).

### EXAMPLE

A clinical study was conducted on a population of 33 idiopathic infertile women with at least one cycle of unsuccessful in vitro fertilization and who received a dose of 2-4 capsules per day of a supplement comprising:
125 mg papain;
215 mg bromelain
131.5 mg curcuma; and
1 .05 mg piperidine

The results in terms of effectiveness of the treatment showed that:
- 100% of the subjects were totally negative for TNF alpha and CATF;
- 31 .8% tested negative for GLY;
- 77.8% tested negative for TOS;

This means that the immune dysfunction is efficiently corrected by the supplemental treatment with the composition according to the invention.

During the study, an assessment was also made of the pregnancy rate in the subsequent in vitro fertilization cycle. The results indicated that the percentage of success of the in vitro fertilization cycle rose from 4.75% to 41 % after treatment with the composition according to the invention.

This means that the immune dysfunction identified is efficiently corrected by the supplemental treatment with the composition according to the invention with a positive influence on the success of the in vitro fertilization cycle.

## Claims

1. A composition for use in the treatment of immune-mediated female infertility, comprising:
- bromelain, in an amount of at least 215 mg by weight per single unit of administration;
- at least one substance capable of exerting an antioxidant action, selected from among papain, curcumin and mixtures thereof, wherein said antioxidant substance comprises, per single unit of administration:
- papain, in an amount of at least 125 mg by weight; and/or
- curcumin, in an amount of at least 131.5 mg by weight, **characterized in that** it further comprises:
- a substance facilitating penetration through a cell membrane; said substance being piperidine.

2. The composition for use according to any one of claim 1, wherein the total mass of a single unit of administration of said composition has an overall weight comprised between 0.40 grams and 1 gram, preferably comprised between 0.45 grams and 0.8 grams.

3. The composition for use according to claim 2, wherein said curcumin is present in phytosome form, said phytosome form comprising curcumin associated with soy phospholipids and/or phosphatidylcholine.

4. The composition for use according to any one of claims 1-3, further comprising at least one substance multiplying the absorption and bioavailability of said antioxidant substance, wherein said at least one absorption and bioavailability multiplying substance is selected from among membrane phospholipids and wherein said membrane phospholipids are present in a ratio of 5:1 by weight relative to said curcumin in said single unit of administration.

5. The composition for use according to any one of claims 1-4, further comprising at least one enzyme belonging to the hydrolase family and capable of exerting a fibrinolytic and/or proteolytic action.

6. The composition for use according to any one of claims 1-5, wherein the bromelain and/or said at least one substance capable of exerting an antioxidant action and/or said at least one penetration facilitating substance are present, in a single administration unit, in micrometric and/or nanometric granular form, said nanometric granular form preferably having an average diameter comprised between 0.01 µm and 2 µm, preferably comprised between 0.3 µm and 1.5 µm.

7. The composition for use according to claim 1, comprising:
- curcuma, preferably as a dry extract;
- papain;
- piperidine, preferably as a dry black pepper extract; and
- bromelain, preferably 1,200 GDU

8. The composition for use according to claim 7, comprising:
- 20.0-30.0% by weight, preferably 26.0 - 29.0%, more preferably approximately 27.8%, of curcuma, preferably as a dry extract;
- 20.0-30.0% by weight, preferably 25.0-28.0%, more preferably approximately 26.5% of papain;
- 0.1-0.3% by weight, preferably approximately 0.2%, of piperidine, preferably as a dry black pepper extract; and
- 40.0-50.0% by weight, preferably 43.0 - 47.0%, more preferably approximately 45.5%, of bromelain, preferably bromelain 1,200 GDU.

9. The composition for use according to claim 7 or 8, wherein curcuma, papain, piperidine and bromelain are present in the following ratios by weight:
- papain/piperidine 110-160;
- bromelain/piperidine 175-225;
- curcuma/piperidine 100-130.

10. A food supplement for use in the treatment of immune-mediated female infertility comprising the composition according to any one of claims 1 to 9 and at least one physiologically acceptable excipient.

11. The composition for use according to any one of claims 1-9 and/or the supplement for use according to claim 10 in the form of tablets or capsules, preferably gastro-resistant capsules.

## Patentansprüche

1. Zusammensetzung zur Verwendung bei der Behandlung von immunvermittelter weiblicher Unfruchtbarkeit, umfassend:
- Bromelain, in einer Menge von mindestens 215 mg Gewicht pro Verabreichungseinheit;
- mindestens eine Substanz, die in der Lage ist, eine antioxidative Wirkung auszuüben, ausgewählt aus Papain, Curcumin und Mischungen davon, wobei die antioxidative Substanz pro einzelner Verabreichungseinheit umfasst:
- Papain, in einer Menge von mindestens 125 mg Gewicht; und/oder
- Curcumin, in einer Menge von mindestens 131,5 mg Gewicht,
**dadurch gekennzeichnet, dass** sie ferner umfasst:
- eine Substanz, die das Durchdringen einer Zellmembran erleichtert, wobei diese Substanz Piperidin ist.

2. Zusammensetzung zur Verwendung nach einem der Ansprüche 1, wobei die Gesamtmasse einer einzelnen Verabreichungseinheit der Zusammensetzung ein Gesamtgewicht zwischen 0,40 Gramm und 1 Gramm, vorzugsweise zwischen 0,45 Gramm und 0,8 Gramm, aufweist.

3. Zusammensetzung zur Verwendung nach Anspruch 2, wobei das Curcumin in phytosomaler Form vorliegt, wobei die phytosomale Form Curcumin in Verbindung mit Sojaphospholipiden und/oder Phosphatidylcholin umfasst.

4. Zusammensetzung zur Verwendung nach einem der Ansprüche 1-3, ferner umfassend mindestens eine Substanz, die die Absorption und Bioverfügbarkeit der antioxidativen Substanz vervielfacht, wobei die mindestens eine die Absorption und Bioverfügbarkeit vervielfachende Substanz aus Membranphospholipiden ausgewählt ist und wobei die Membranphospholipide in der einzelnen Verabreichungseinheit in einem Gewichtsverhältnis von 5:1 relativ zu dem Curcumin vorhanden sind.

5. Zusammensetzung zur Verwendung nach einem der Ansprüche 1-4, ferner umfassend mindestens ein Enzym, das zur Familie der Hydrolasen gehört und eine fibrinolytische und/oder proteolytische Wirkung ausüben kann.

6. Zusammensetzung zur Verwendung nach einem der Ansprüche 1-5, wobei das Bromelain und/oder die mindestens eine Substanz, die eine antioxidative Wirkung ausüben kann, und/oder die mindestens eine das Durchdringen erleichternde Substanz in einer einzigen Verabreichungseinheit in mikrometrischer und/oder nanometrischer körniger Form vorliegen, wobei die nanometrische körnige Form vorzugsweise einen durchschnittlichen Durchmesser zwischen 0,01 µm und 2 µm, vorzugsweise zwischen 0,3 µm und 1,5 µm aufweist.

7. Zusammensetzung zur Verwendung nach Anspruch 1, umfassend:
- Kurkuma, vorzugsweise als Trockenextrakt;
- Papain;
- Piperidin, vorzugsweise als Trockenextrakt aus schwarzem Pfeffer; und
- Bromelain, vorzugsweise 1.200 GOU

8. Zusammensetzung zur Verwendung nach Anspruch 7, umfassend:
- 20,0 - 30,0 Gew.-%, vorzugsweise 26,0 - 29,0 Gew.-%, mehr bevorzugt etwa 27,8 Gew.-% Kurkuma, vorzugsweise als Trockenextrakt;
- 20,0 - 30,0 Gew.-%, vorzugsweise 25,0 - 28,0 Gew.-%, mehr bevorzugt etwa 26,5 Gew.-% Papain;
- 0,1 - 0,3 Gew.-%, vorzugsweise etwa 0,2 % Piperidin, vorzugsweise als Trockenextrakt aus schwarzem Pfeffer; und
- 40,0 - 50,0 Gew.-%, vorzugsweise 43,0 - 47,0 Gew.-%, mehr bevorzugt etwa 45,5 Gew.-% Bromelain, vorzugsweise Bromelain 1.200 GOU.

9. Zusammensetzung zur Verwendung nach Anspruch 7 oder 8, wobei Kurkuma, Papain, Piperidin und Bromelain in den folgenden Gewichtsverhältnissen vorhanden sind:
- Papain/Piperidin 110 - 160;
- Bromelain/Piperidin 175 - 225;
- Kurkuma/Piperidin 100 - 130.

10. Nahrungsergänzungsmittel zur Verwendung bei der Behandlung von immunvermittelter weiblicher Unfruchtbarkeit, umfassend die Zusammensetzung nach einem der Ansprüche 1 bis 9 und mindestens einen physiologisch akzeptablen Trägerstoff.

11. Zusammensetzung zur Verwendung nach einem der Ansprüche 1-9 und/oder das Ergänzungsmittel zur Verwendung nach Anspruch 10 in Form von Tabletten oder Kapseln, vorzugsweise magensaftresistenten Kapseln.

## Revendications

1. Composition pour une utilisation dans le traitement de l'infertilité féminine auto-immune, comprenant :
- de la bromélaïne, dans une quantité d'au moins 215 mg en poids par unité unique d'administration ;
- au moins une substance capable d'exercer une action antioxydante, sélectionnée parmi la papaïne, la curcumine et leurs mélanges, dans laquelle ladite substance antioxydante comprend, par unité unique d'administration :
- de la papaïne, dans une quantité d'au moins 125 mg en poids ; et/ou
- de la curcumine, dans une quantité d'au moins 131,5 mg en poids, **caractérisée en ce qu'**elle comprend en outre :
- une substance facilitant la pénétration à travers une membrane cellulaire ; ladite substance étant la pipéridine.

2. Composition pour une utilisation selon l'une quelconque de la revendication 1, dans laquelle la masse totale d'une unité unique d'administration de ladite composition possède un poids global compris entre 0,40 gramme et 1 gramme, de préférence compris entre 0,45 gramme et 0,8 gramme.

3. Composition pour une utilisation selon la revendication 2, dans laquelle ladite curcumine est présente sous forme de phytosome, ladite forme de phytosome comprenant de la curcumine associée à des phospholipides de soja et/ou de la phosphatidylcholine.

4. Composition pour une utilisation selon l'une quelconque des revendications 1 à 3, comprenant en outre au moins une substance multipliant l'absorption et la biodisponibilité de ladite substance antioxydante, dans laquelle ladite au moins une substance multipliant l'absorption et la biodisponibilité est sélectionnée parmi les phospholipides membranaires et dans laquelle lesdits phospholipides membranaires sont présents selon un rapport de 5:1 en poids par rapport à ladite curcumine dans ladite unité unique d'administration.

5. Composition pour une utilisation selon l'une quelconque des revendications 1 à 4, comprenant en outre au moins une enzyme appartenant à la famille des hydrolases et capable d'exercer une action fibrinolytique et/ou protéolytique.

6. Composition pour une utilisation selon l'une quelconque des revendications 1 à 5, dans laquelle la bromélaïne et/ou ladite au moins une substance capable d'exercer une action antioxydante et/ou ladite au moins une substance facilitant la pénétration sont présentes, dans une unité unique d'administration, sous forme granulaire micrométrique et/ou nanométrique, ladite forme granulaire nanométrique ayant de préférence un diamètre moyen compris entre 0,01 µm et 2 µm, de préférence compris entre 0,3 µm et 1,5 µm.

7. Composition pour une utilisation selon la revendication 1, comprenant :
- du curcuma, de préférence sous la forme d'un extrait sec ;
- de la papaïne ;
- de la pipéridine, de préférence sous la forme d'un extrait sec de poivre noir ; et
- de la bromélaïne, de préférence à 1 200 unités de digestion de gélatine, GDU.

8. Composition pour une utilisation selon la revendication 7, comprenant :
- de 20,0 à 30,0 % en poids, de préférence de 26,0 à 29,0 %, plus préférentiellement approximativement 27,8 %, de curcuma, de préférence sous la forme d'un extrait sec ;
- de 20,0 à 30,0 % en poids, de préférence de 25,0 à 28,0 %, plus préférentiellement approximativement 26,5 % de papaïne ;
- de 0,1 à 0,3 % en poids, de préférence approximativement 0,2 %, de pipéridine, de préférence sous la forme d'un extrait sec de poivre noir ; et
- de 40,0 à 50,0 % en poids, de préférence de 43,0 à 47,0 %, plus préférentiellement approximativement 45,5 %, de bromélaïne, de préférence de la bromélaïne à 1 200 GDU.

9. Composition pour une utilisation selon la revendication 7 ou 8, dans laquelle le curcuma, la papaïne, la pipéridine et la bromélaïne sont présents selon les rapports en poids suivants :
- papaïne/pipéridine 110 à 160 ;
- bromélaïne/pipéridine 175 à 225 ;
- curcuma/pipéridine 100 à 130.

10. Complément alimentaire pour une utilisation dans le traitement de l'infertilité féminine auto-immune comprenant la composition selon l'une quelconque des revendications 1 à 9 et au moins un excipient physiologiquement acceptable.

11. Composition pour une utilisation selon l'une quelconque des revendications 1 à 9 et/ou complément pour une utilisation selon la revendication 10 sous la forme de comprimés ou de gélules, de préférence de gélules gastro-résistantes.
